# EUROPEAN PATENT SPECIFICATION

(11) **EP 0 425 257 B1**
(45) Date of publication and mention of the grant of the patent: **30.11.1994**
(21) Application number: 90311634.1
(22) Date of filing: 24.10.1990
(51) Int. Cl.: A61M 1/10, F04D 13/02

(54) **Centrifugal blood pump**
Zentrifugalblutpumpe
Pompe de sang centrifuge

(30) Priority: 24.10.1989 US 426102
(43) Date of publication of application: 02.05.1991
(73) Proprietor: MINNESOTA MINING AND MANUFACTURING COMPANY, St. Paul, Minnesota 55133-3427 (US)
(72) Inventor: Clausen, Earl W. c/o Minnesota Mining & Manuf.Comp, St.Paul, Minnesota 55133-3427 (US); Hubbard, Lloyd C. c/o Minnesota Mining & Manuf.Co., St.Paul, Minnesota 55133-3427 (US)
(74) Representative: Baillie, Iain Cameron

(56) References cited:
- WO-A-89/07427
- DE-A- 1 728 462
- DE-B- 2 200 599
- US-A- 3 411 450
- US-A- 3 771 910
- US-A- 4 643 641
- ENGINEERS DIGEST, vol. 32, no. 7, July 1971, page 41; LAING PHYSIKATISCH-TECKNISCHES FORSCHUNGSINSTITUT: "Magnetic suspension and shaftless drive system for pumps"

## Description

The invention relates to centrifugal blood pumps, and in particular to a centrifugal blood pumping unit that is adapted to be releasably coupled to a magnetic drive device.

Centrifugal pumps have been used for many years to pump a wide variety of different fluid materials. In general, a centrifugal pump includes a pump housing enclosing a pumping chamber therein, an inlet aligned with a rotational axis of the pump, an outlet adjacent the periphery of the pumping chamber, an impeller mounted within the pumping chamber for rotation about the axis, and a drive source communicating with the impeller. The impeller and drive source have several possible configurations. In one configuration, the impeller is mounted on a drive shaft which extends outside the pumping chamber to a rotational drive source. In another configuration, the pump housing encloses two chambers, one containing a magnetic rotor and the other containing the pumping chamber and impeller. The impeller and rotor are connected by a drive shaft. Seals are used to isolate the two chambers. A magnetic drive source communicates with the rotor to rotate the impeller within the pumping chamber. In still another configuration, the impeller is suspended within the pumping chamber by a magnetic means formed within the pump housing. Examples of these centrifugal pumps are shown in the following U.S. Patents: Kletschka et al. U.S. Pat. No. 3,864,055; Rafferty et al U.S. Pat. No. 3,647,324; and Olsen et al U.S. Pat. No. 4,688,998.

In recent years, centrifugal pumps have been used extensively for pumping blood during open heart surgery. The pumping of blood requires great care to avoid any damage to the red corpuscles, or any of the other constituents of blood. Any practical blood pump useful as part of heart/lung bypass equipment during open heart surgery must deliver the requisite flow volumes under pressure, without damaging the blood being pumped.

In many prior centrifugal pumps, and in particular in a centrifugal pump for pumping liquids such as blood, a fluid tight seal between the drive shaft and the housing is an important factor in the performance of the pump. Friction at the seal produces heat which, if not dissipated, can damage both the components of the pump and the blood being pumped. Also, the rotation of the impeller can lead to generation of an air bubble surrounding the shaft. This air bubble tends to seek the smallest shaft diameter, which typically is adjacent the drive shaft seal. In some of the prior art pumps, the area adjacent the drive shaft seal has also been a relatively stagnant or low flow area in terms of fluid flow within the pumping chamber. The air bubble tends to insulate the seal from the flow of the fluid within the pumping chamber, thus decreasing the dissipation of heat generated by friction at the seal surface.

It is often the case that blood pumps are used only once. After a single use, the portions of the pump which contact the blood must either be disposed of or its constituent parts must be sterilized. A centrifugal blood pump comprising a minimal number of parts is desirable to reduce costs and improve reliability.

U.S. Patent Nos. 4,507,048; 4,589,822; 4,606,698; 4,643,641 and 4,898,518 describe various centrifugal blood pumps.

Additional examples of centrifugal pumps are shown in U.S. Patent Nos. 3,354,833; 3,411,450; 3,645,650; 3,771,910; 3,762,839; 3,838,947 and 4,352,646, as well as in Japanese Kokoku No. 43(1968)/17206 and German Offenlegungsschrift Nos. 1,728,462 and 2,048,286. The Japanese Kokoku No. 43/17206 describes a centrifugal pump in which a pump runner is magnetically coupled with a group of drive magnets. The pump runner is positioned within a pump housing, and rotatable about a pivot or thrust bearing supported by struts near the inlet of the pump housing. The pump runner carries a group of magnets that are driven by the drive magnets. When rotation of the drive magnets of that pump is stopped, the pump runner contacts the bottom plate due to the force of attraction between the drive and driven magnets, and a gap is formed between the pivot bearing and the runner.

### Summary of the Invention

According to a first aspect of the present invention there is provided a disposable pumping unit adapted to be releasably mounted on a magnetic drive means for pumping blood, the pumping unit comprising:
a pump housing having a pumping chamber therein, and an inlet and an outlet communicating with the pumping chamber;
a bearing supported in the pumping chamber;
an impeller positioned within the pumping chamber the impeller having a hub rotatably supported on the bearing for rotation about a central axis; and
magnetic means carried by the impeller within the pumping chamber adapted to be magnetically coupled with a magnetic drive means to rotate the impeller, and thereby pump fluid through the pumping unit characterized by:-
said impeller having a plurality of openings configured to direct blood flow along the bearing.

According to a second aspect of the present invention there is provided a centrifugal pump for pumping blood, comprising:
a disposable pumping unit comprising:
a pump housing having a pumping chamber therein, and having an inlet and an outlet communicating with the pumping chamber;
a bearing supported in the pumping chamber, the bearing defining a central axis;
an impeller positioned within the pumping chamber and supported on the bearing for rotation about the central axis, the impeller having a hub;
magnetic means carried by the impeller; and
magnetic drive means for releasable connection to the pump housing to communicate with the magnetic means carried by the impeller and thereby to rotate the impeller within the pumping chamber characterised by:
said impeller having a plurality of openings configured to direct blood flow along the bearing.

There is thus disclosed a centrifugal pumping unit for pumping blood, which is disposable after one use. The pumping unit is adapted to be releasably mounted on a magnetic drive means.

Generally, the pumping unit comprises a pump housing enclosing a pumping chamber therein, a bearing supported in the pumping chamber, and an impeller positioned within the pumping chamber. The pump housing has an inlet and an outlet communicating with the pumping chamber. The impeller is supported at its hub by the bearing for rotation about a central axis defined by the bearing. A plurality of openings, which are configured to expose the bearing to fluid, are provided in the impeller. A magnetic means (e.g., at least one magnet) is carried by the impeller, and is adapted to be magnetically coupled with the magnetic drive means to rotate the impeller, thereby pumping fluid through the pumping unit..

In one preferred embodiment, the bearing is a ball-shaped pivot bearing made, for example, from hardcoated aluminum to provide sufficient heat dissipation. The impeller carries an annular magnetic ring about its circumference. The magnetic ring has a plurality of magnetic poles and is positioned such that the magnetic lines of force are substantially directed toward the bearing and the central axis. The resulting unbalanced forces on the impeller hub are generally parallel to the central axis in a "downward" direction from the hub toward the bearing, therefore stabilizing the rotation of the impeller about the central axis.

The centrifugal pump preferably includes a magnetic drive device releasably connected to the disposable pumping unit. The magnetic drive device includes a rotor having a plurality of drive magnets spaced annularly about its circumference. The drive magnets are positioned such that their magnetic lines of force align with and are generally parallel to the magnetic lines of force of the magnetic ring carried by the impeller within the pumping chamber. The drive magnets communicate with the magnetic ring carried by the impeller, and thereby rotate the impeller within the pumping chamber as the rotor of the magnetic drive device is rotated.

### Brief Description of the Drawings

Fig. 1 is a perspective view of a centrifugal blood pump of the present invention.

Fig. 2A is an exploded view of the pump of Fig. 1 showing the disposable pumping unit detached from the magnetic drive device.

Fig. 2B is an exploded view of the centrifugal blood pump of Figs. 1 and 2A.

Fig. 3 is a plan view of the pumping unit of Figs. 1, 2A and 2B removed from the magnetic drive means.

Fig. 4 is a transverse section of the pump taken along line 4-4 of Fig. 3 and includes the magnetic drive device of Figs. 1, 2A and 2B.

Fig. 5 is a view similar to Fig. 4 of an alternate embodiment having a low profile and an alternate stator arrangement.

Fig. 6 is an alternate embodiment having a low profile and stator arrangement similar to the one shown in Fig. 4.

Fig. 7 is a fragmentary detail of an alternate bearing arrangement.

Fig. 8 is a partial sectional view of an embodiment with an alternate impeller arrangement.

### Detailed Description of the Preferred Embodiments

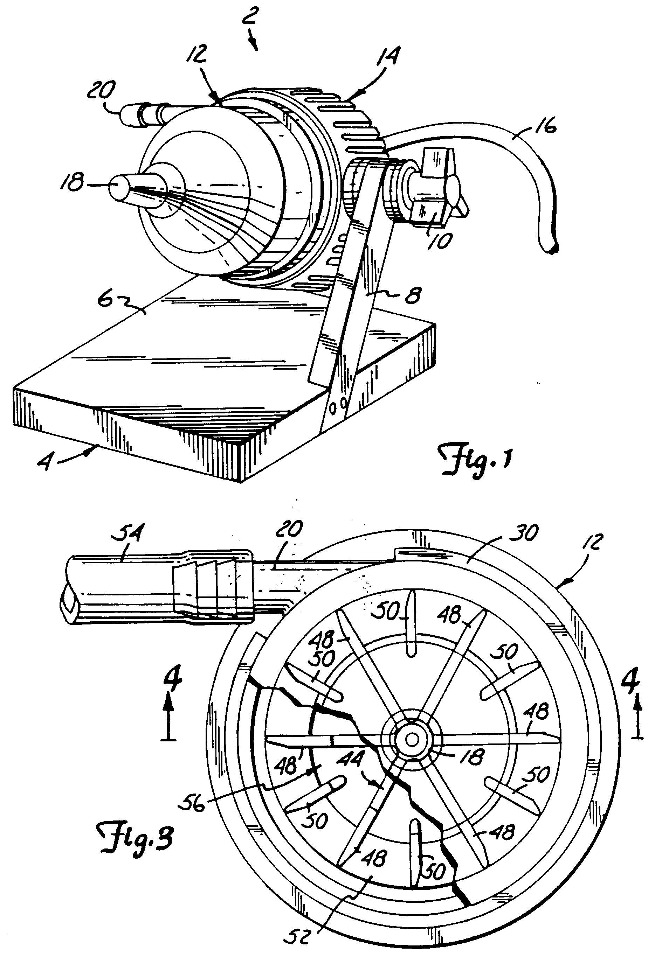

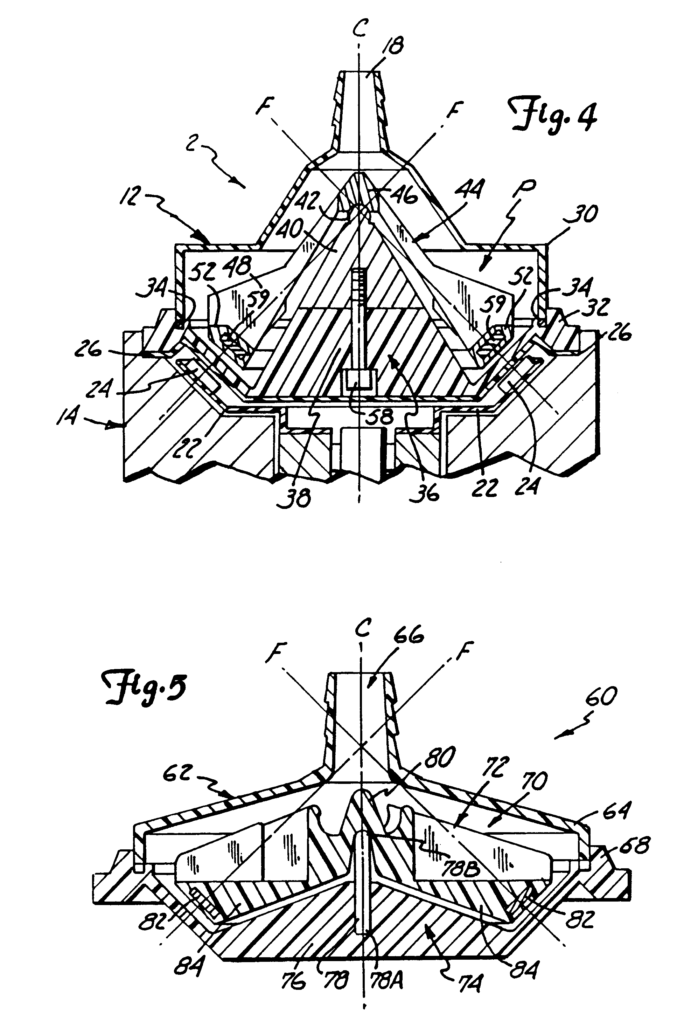

Figs. 1-4 show one embodiment of the centrifugal blood pump of the invention. Figs. 5-8 illustrate alternate embodiments.

Fig. 1 shows a perspective view of a centrifugal blood pump 2. Pump 2 is mounted on stand 4 (formed by base 6, support arm 8 and locking nut 10) and comprises a disposable pumping unit 12 and a magnetic drive device 14. Disposable pumping unit 12 is releasably connected to magnetic drive device 14. Electric cord 16 connects magnetic drive device 14 to an electric power source (not shown) and thereby provides electric excitation to magnetic drive device 14. During pump operation, blood enters disposable pumping unit 12 through inlet 18 and is pumped out through outlet 20.

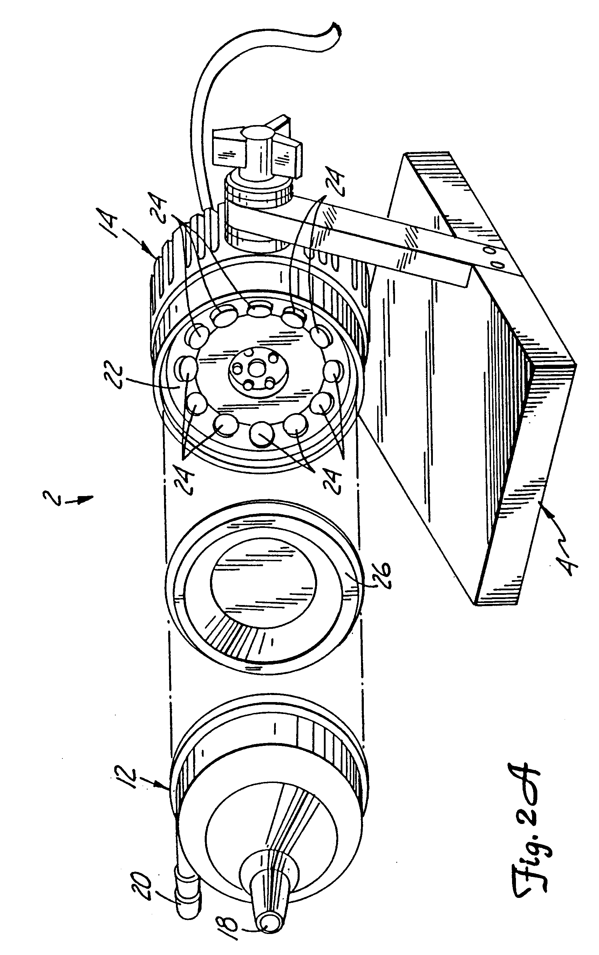

Figs. 2A and 2B are exploded views of the centrifugal pump shown in Fig. 1. Fig. 2A shows the centrifugal pump of Fig. 1 with disposable pumping unit 12 detached from magnetic drive means or device 14. The magnetic drive device 14 includes a rotor 22 having a plurality of circumferentially spaced drive magnets 24. A protective plate 26 is positioned adjacent the drive magnets 24 and is supported by the face of the magnetic drive device 14. The protective plate 26 isolates rotor 22 from foreign objects and contaminants which could impede the operation of magnetic drive device 14.

As shown in Fig. 2B, the disposable pumping unit 12 comprises a pump housing (formed by housing cap 30 and housing base 32) enclosing a pumping chamber P therein (shown in Fig. 4). Housing cap 30 includes inlet 18 and outlet 20, and is preferably transparent so that operation of pump 2 can be visually monitored. The inlet 18 is aligned with a central axis C and the outlet 20 is positioned at the periphery of housing cap 30. An annular seal 34 is placed between housing cap 30 and housing base 32 to provide a fluid-tight seal for the pumping chamber P.

The disposable pumping unit 12 also includes a stator 36 having a proximal end and a distal end. The proximal end is secured to housing base 32 and the distal end extends into the pumping chamber P. Stator 36 defines the central axis C and is generally conically shaped to reduce the stagnation of fluid near the central axis C. The stator 36 comprises two sections: a proximal section 38 and a distal section 40. Proximal section 38 is shaped as a frustal cone which can either be secured to or formed integral with housing base 32. Distal section 40 is cone shaped and secured to proximal section. Stator 36, in an alternate embodiment, can be formed as one piece. As used herein the word "stator" refers to a stationary member around which a rotating member, such as the impeller 44, can rotate.

A bearing 42 is formed integrally with the distal end of the stator 36. The bearing 42, alternatively, can be formed separately and then secured to the distal end of the stator 36. The bearing 42 is shown as a ball-shaped pivot bearing but any one of a number of conventional bearing types could be employed. The bearing 42 is generally aligned with the central axis C. The bearing 42 is preferably made from a material having sufficient heat dissipation qualities, such as hard-coated aluminum. The distal section 40 of the stator 36 is also preferably made from material having good heat dissipation qualities. Heat dissipation is very important near the bearing 42 to protect both the bearing 42 and the blood being pumped through the pumping chamber P.

A rotator or impeller 44 is positioned within the pumping chamber and is supported on bearing 42 for rotation about the central axis C. In this embodiment, the impeller 44 is configured to have a generally conical shape. The impeller 44 has a hub 46 aligned with the central axis C for engagement with bearing 42. The impeller 44 includes long blades 48, short blades 50, and a circular flange 52. The long blades 48 are attached at their inner ends to the impeller hub 46. The flange 52 is attached to and is supported by the long blades 48. The short blades 50 are supported by the flange 52. In the particular embodiment shown in Figures 2A and 2B, the long and short blades 48 and 50 are alternately spaced about the circumference of the impeller 44. Large diameter impellers require a greater number of blades in order to achieve pumping efficiency. By use of short blades 50 supported by flange 52, the impeller 44 achieves pumping efficiency while retaining a small hub diameter, since only long blades 48 are attached to hub 46. As shown in figure 2B, openings are formed between adjacent long blades 48 that are believed to allow fluid to flow over the distal end 40 of the stator 36 and bearing 42. The impeller 44 carries magnetic means 59 (shown in Fig. 4) on or within flange 52 such that the magnetic means is axially displaced from bearing 42.

Magnetic drive device 14 includes drive magnets 24 which communicate with magnetic means 59 carried by the flange 52 of the impeller 44 to rotate the impeller 44 within the pumping chamber P. The magnetic means 59 carried by the impeller 44 and the drive magnets 24 carried by rotor 22 must have enough attraction strength to achieve the desired operation of centrifugal blood pump 2. It is preferable to have drive magnets 24 be stronger than magnetic means 59 carried by the impeller 44 so that less expensive magnets may be used within the disposable pumping unit 12.

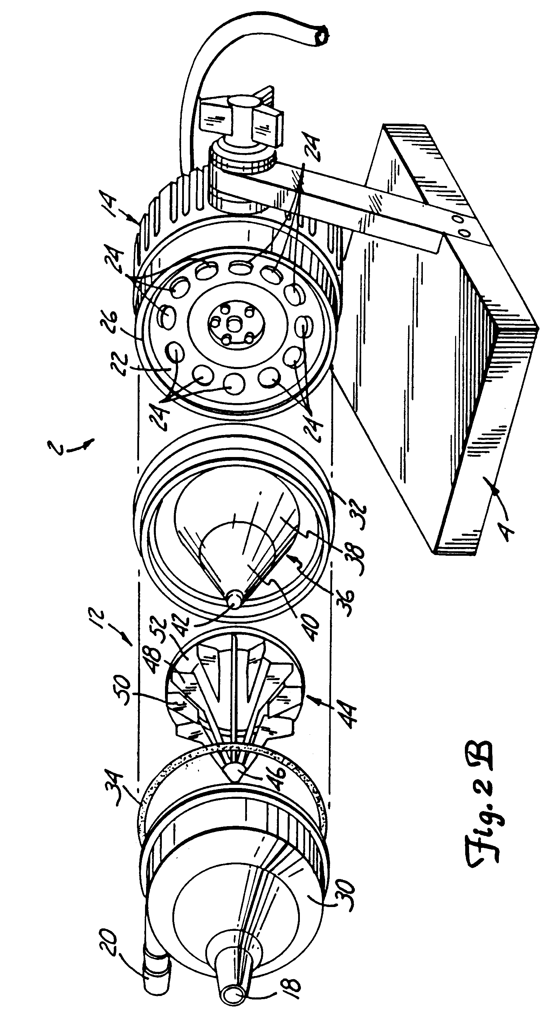

Fig. 3 is a plan view of the disposable pumping unit 12 shown in Figs. 1, 2A and 2B. The transparent housing cap 30 is shown with the inlet 18 positioned along the central axis C and the outlet 20 positioned at the periphery. A tube 54 can be fitted to housing cap 30 at the outlet 20 for transferring fluid from the pumping chamber 56 to a destination. The impeller 44 is shown with long blades 48, short blades 50 and flange 52.

Fig. 4 is a transverse section of the centrifugal pump 2 shown in Figs. 1, 2A and 2B. The centrifugal pump 2 comprises the disposable pumping unit 12 and the magnetic drive device 14. The disposable pumping unit 12 includes a housing cap 30 (with inlet 18), housing base 32, annular seal 34, stator 36, screw 58, and impeller 44. The stator 36 comprises a proximal section 38 and a distal section 40 which are secured together by a screw 58. In this embodiment, the proximal section 38 is formed integrally with the housing base 32. The bearing 42 is formed integrally with the distal end of the stator 36, and supports the hub 46 of the impeller 44. The impeller 44 is shown carrying the magnetic means 59 on the flange 52. The magnetic means 59 is preferably a magnetic ring having a plurality of magnetic poles (not shown) and is commercially available. Alternatively, a plurality of circumferentially spaced magnets can be carried by the flange 52. The magnetic drive device 14 comprises a protective plate 26 and drive magnets 24 carried on the rotor 22.

Fig. 4 also shows the positional relationship between the magnetic means 59 carried by the impeller 44 and the drive magnets 24 carried by the rotor 22. The magnetic lines of force F of the magnetic means 59 are substantially directed toward the bearing 42 and the central axis C. The drive magnets 24 are positioned such that their magnetic lines of force F generally align with and are parallel to the magnetic lines of force F generated by the magnetic means 59 carried by the impeller 44. The resulting unbalanced forces on the impeller 44 generally align with the central axis C and are in a direction from impeller hub 46 toward bearing 42. With this magnet orientation, the balanced forces stabilize rotation of the impeller 44 about the central axis C and hold the impeller 44 against the bearing 42.

Fig. 5 shows an alternate embodiment of a disposable pumping unit 60 having a lower profile than shown in Fig. 4. This embodiment includes a pump housing 62, housing cap 64 with inlet 66, housing base 68, pumping chamber 70, impeller 72, and stator 74. The outlet of the housing 62 is not shown in Fig. 5, but is positioned similarly to outlet 20 shown in Fig. 1. Fig. 5 not only shows a low profile pumping unit, but also shows stator 74 as an alternate arrangement to stator 36 shown in Fig. 4. Stator 74 comprises a stator base 76 (formed integrally with the housing base 68) and a spindle 78, which generally replace bearing 42 and distal section 40 of stator 36. Spindle 78 has first and second opposite ends 78A and 78B. The first end 78A is secured to the stator base 76 of stator 74; and the second end 78B extends into the pumping chamber 70 and is rounded for use as a bearing (at 78B).

The impeller 72 of figure 5 is similar to the impeller 44 described in Fig. 4 but it too has a lower profile. The impeller 72 has a different hub configuration 80 to accommodate the lower profile without sacrificing impeller blade area. The hub 80 is preferably made from hardcoated aluminum or similar heat dissipating material. The impeller 72 carries a magnetic means 82 on the flange 84 of the impeller 72 such that the magnetic lines of force F are generally directed toward the bearing at the second end 78B of the spindle 78 and the central axis C so that rotation of the impeller 72 is stabilized about the central axis C. The lower profile pumping unit 60 reduces the volume of the pumping chamber 70 while maintaining pumping efficiency. This reduces the amount of blood necessary to prime the pump before the start of operation.

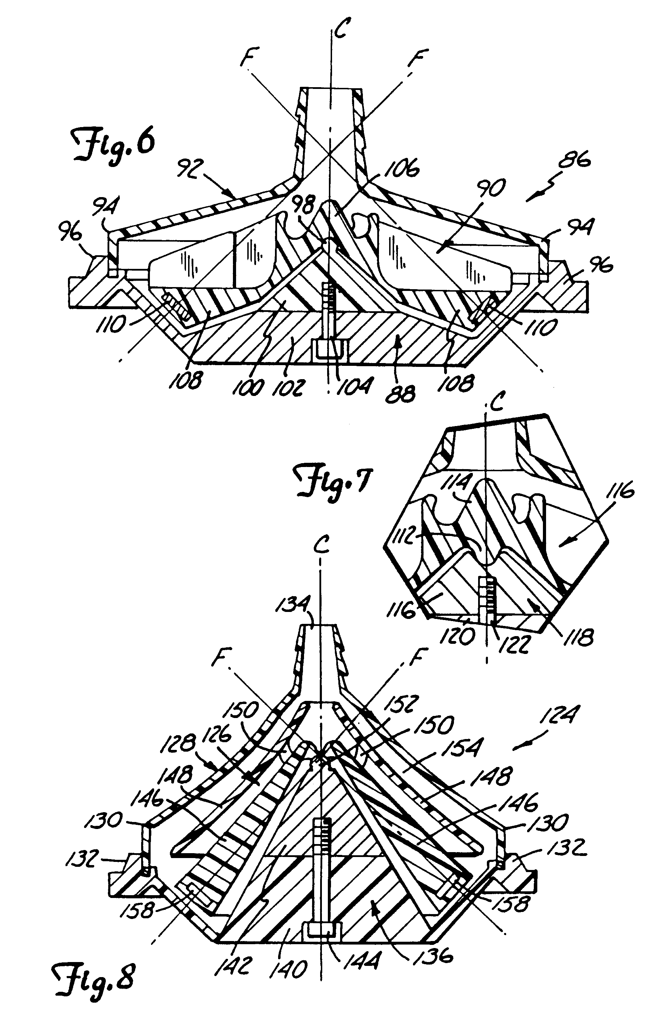

Fig. 6 shows another low profile disposable pumping unit embodiment 86. The pumping unit 86 comprises a stator 88, impeller 90, and pump housing 92. Pump housing 92 includes housing cap 94 and housing base 96. A ball-shaped pivot bearing 98 is used instead of the spindle of Fig. 5. The housing base 68 and stator 74 of Fig. 5 have been replaced with the housing base 96 and stator 88. The stator 88 has a similar configuration to stator 36 shown in Fig. 4 but has a lower profile. The stator 88 comprises a distal section 100, proximal section 102, and screw 104. The stator 88 defines central axis C. The impeller 90 is similar to the impeller 72 illustrated in Fig. 5 and includes a hub 106, flange 108, and magnetic means 110. The magnetic means 110 creates magnetic lines of force F. In this embodiment, the ball-shaped pivot bearing 98 is formed integrally with the distal section 100 of the stator 88, and the distal section 100 and bearing 98 are preferably formed of hardcoated aluminum or similar heat dissipating material.

The pumping unit 86 of Fig. 6 is shown with an alternate bearing arrangement in the partial sectional view of Fig. 7. A convex bearing 112 is formed integrally with the hub 114 of the impeller 116. The convex bearing 112 rests on the concave bearing surface 117 of the distal section 119 of stator 118 for rotation about the central axis C. Distal section 116 is secured to proximal section 120 by screw 122. The convex bearing 112 can also be formed as a separate element and secured to the impeller hub (not shown).

Fig. 8 shows an alternate embodiment of a disposable pumping unit 124 having an alternate impeller configuration 126. The pumping unit 124 includes a pump housing 128, housing cap 130, housing base 132, inlet 134, an outlet (not shown) located at the periphery of housing 128, stator 136, and impeller 126. The stator 136 is similar to the stator 36 in Fig. 4 and comprises a proximal section 140, a distal section 142, and a screw 144. The impeller 126 comprises first and second concentric rotator cones 146 and 148 secured together with a plurality of struts 150. The first rotator cone 146 is supported by bearing 152 for rotation of the first and the second rotator cones 146 and 148 about central axis C. The first and second cones 146 and 148 each have an opening at the central axis C allowing fluid entering the pumping chamber 154 through the inlet 134 to increase heat dissipation by lubricating the bearing 152. This fluid flow path also limits the stagnation of fluid near the wall of the stator 136. The first rotator cone 146 carries a magnetic means 158 about its circumference and is oriented such that the magnetic lines of force F are substantially directed toward the bearing 152 and the central axis C.

The invention provides a centrifugal blood pump having a minimal number of parts requiring disposal or sterilization. The drive shaft seals of prior centrifugal blood pumps have been eliminated, which increases reliability of the pump of this invention. Reliability is further increased by positioning the bearing, as shown in Fig. 4, near the inlet, and by providing openings through the impeller that direct blood flow along the bearing. This increases blood flow near the bearing and thereby reduces heat build-up on the bearing and on the blood near the surface of the bearing. Heat damage to the blood being pumped is therefore also reduced.

The reduced parts count of the disposable pumping unit along with its simplistic design lowers the relative cost of centrifugal blood pumps with respect to prior centrifugal blood pumps.

## Claims

1. A disposable pumping unit (12; 60; 86; 124) adapted to be releasably mounted on a magnetic drive means (14) for pumping blood, the pumping unit (12; 60; 86; 124) comprising:
a pump housing (30 and 32; 62; 92; 128) having a pumping chamber (P; 70) therein, and an inlet (18; 66; 134) and an outlet (20) communicating with the pumping chamber (P; 70);
a bearing (42; 78B; 98; 152) supported in the pumping chamber (P; 70);
an impeller (44; 72; 90; 126) positioned within the pumping chamber (P; 70), the impeller (44; 72; 90; 126) having a hub (46; 80; 106) rotatably supported on the bearing (42; 78B; ;98; 152) for rotation about a central axis (C); and
magnetic means (59; 82; 110; 158) carried by the impeller (44; 72; 90; 126) within the pumping chamber (P; 70) adapted to be magnetically coupled with a magnetic drive means (14) to rotate the impeller (44; 72; 90; 126), and thereby pump fluid through the pumping unit (12; 60; 86; 124) characterized by:-
said impeller having a plurality of openings configured to direct blood flow along the bearing (42; 78B; 98; 152).

2. A centrifugal pump (2) for pumping blood, comprising:
a disposable pumping unit (12; 60; 86; 124) comprising:
a pump housing (30 and 32; 62; 92; 128) having a pumping chamber (P; 70) therein, and having an inlet (18; 66; 134) and an outlet (20) communicating with the pumping chamber (P; 70);
a bearing (42; 78B; 98; 152) supported in the pumping chamber (P; 70), the bearing (42; 78B; 98; 152) defining a central axis (C);
an impeller (44; 72; 90; 126) positioned within the pumping chamber (P; 70) and supported on the bearing (42; 78B; 98; 152) for rotation about the central axis (C), the impeller (44; 72; 90; 126) having a hub (46; 80; 106);
magnetic means (59; 82; 110; 158) carried by the impeller (44; 72; 90; 126); and
magnetic drive means (14) for releasable connection to the pump housing (32; 62; 92; 128) to communicate with the magnetic means (59; 82; 110; 158) carried by the impeller (44; 72; 90; 126) and thereby to rotate the impeller (44; 72; 90; 126) within the pumping chamber (P; 70) characterised by:
said impeller having a plurality of openings configured to direct blood flow along the bearing (42; 78B; 98; 152).

3. The centrifugal pump (2) according to claim 2 wherein the magnetic drive means (14) comprises:
a rotor (22) positioned adjacent the pump housing (32; 62; 92; 128); and
a plurality of drive magnets (24) annularly spaced about the circumference of the rotor (22) and oriented with the magnetic lines of force (F) generated by the drive magnets (24) aligning with the magnetic lines of force (F) generated by the magnetic means (59; 82; 110; 158) carried by the impeller (44; 72; 90; 126) and intersecting the central axis (C) such that resulting unbalanced forces on the impeller hub (46; 80; 106) are in a direction tending to urge the impeller hub (46; 80; 106) toward the bearing (42; 78B; 98; 152), the resulting unbalanced forces being generally parallel to the central axis (C) thereby stabilizing the rotation of the impeller (44; 72; 90; 126) about the bearing (42; 78B; 98; 152) and the central axis (C).

4. A pumping unit (12; 60; 86; 124) or centrifugal pump (2) according to claims 1, 2 or 3 wherein the bearing (42; 78B; 98; 152) is supported adjacent the inlet (18; 66; 134) for the flow of incoming fluid over the bearing (42; 78B; 98; 152), the bearing (42; 78B; 98; 152) and the inlet (18; 66; 134) being aligned along the central axis (C) of rotation of the impeller (44; 72; 90; 126), the direction along the central axis (C) from the bearing (42; 78B; 98; 152) toward the inlet (18; 66) constituting the upstream direction, the magnetic means (69; 82; 110; 158) forming magnetic lines of force (F) intersecting the central axis (C) of rotation of the impeller (44; 72; 90; 126) at or upstream of the bearing (42; 78B; 98; 152).

5. A pumping unit (12; 60; 86; 124) or centrifugal pump (2) according to claim 4 wherein the pump housing (30 and 32; 62; 92; 128) comprises:
a housing cap (30; 64; 94; 130) having the inlet (18; 66) aligned with the central axis (C) for routing fluid into the pumping chamber (P; 70);
a housing base (32; 68; 96; 132) secured to the housing cap (30; 64; 94; 130) thereby enclosing the pumping chamber (P; 70) therein; and
an annular fluid-tight seal (34) between the housing cap (30; 64; 94; 130) and base (32; 68; 96; 132).

6. A pumping unit (12; 60; 86) or centrifugal pump (2) according to claim 4 wherein the impeller (44; 72; 90) has a periphery and a plurality of radial blades (48) extending to the periphery, the hub (46; 80; 106) of the impeller (44; 72; 90) being positioned between the inlet (18; 66) and the bearing (42; 78B; 98), the radial blades (48) defining the plurality of openings of the impeller (44; 72; 90) that are configured to expose the bearing (42; 78B; 98) to incoming fluid from the inlet (18; 66), the magnetic means (59) forming magnetic lines of force (F) which intersect the central axis (C) of rotation of the impeller (44; 72; 90) such that resulting balanced and unbalanced forces on the impeller (44; 72; 90) stabalize rotation about the bearing (42; 78B; 98) and the central axis (C).

7. A pumping unit (60) or centrifugal pump (2) according to claims 5 or 6 wherein the pumping unit (60) further comprises a stator (74) having a proximal end and a distal end (78B) with the proximal end connected to the pump housing (62) and the distal end (78B) extending into the pumping chamber (70); the stator (74) comprising:
a stator base (76) shaped as a frustal cone with a trapezoidal cross-section along the central axis (C), the stator base (76) having a maximum radial dimension and a minimum radial dimension, the maximum radial dimension defining the proximal end of the stator (74); and
a spindle (78) having first and second opposite ends (78A and 78B) aligned with the central axis (C), the first end (78A) attached to the minimum radial dimension of the stator base (76) and the second end (78B) defining the distal end (78B) of the stator (74);
the bearing (78B) being integrally formed with the second end (78B) of the spindle (78) in alignment with the central axis (C).

8. A pumping unit (12; 86; 124) or centrifugal pump (2) according to claims 4 or 5 wherein the pumping unit (12; 86; 124) further comprises a stator (36; 88; 136) having a proximal end and a distal end with the proximal end connected to the housing base (32; 96; 132) of the pump housing (30 and 32; 92; 128) and the distal end extending into the pumping chamber (P), the stator (36; 88; 136) defining a central axis (C) coaxial with the axis of rotation (C) of the impeller (44; 90; 126), the stator (36; 88; 136) comprising a proximal section (38; 102; 140) of polymeric material and a distal section (40; 100; 142) of hardcoated aluminum, the stator (36; 88; 136) being conically-shaped with the proximal end having a larger radius than the distal end; the bearing (42; 98; 152) being a ball-shaped pivot bearing (42; 98; 152) integrally formed of hardcoated aluminum with the distal section (40; 100; 142) of the stator (36; 88; 136) in alignment with the central axis (C); the impeller (44; 90; 126) being conically-shaped with a maximum radial end adjacent the proximal end of the stator (36; 88; 136) and a minimum radial end supported by the bearing (42; 98; 152).

9. A pumping unit (124) or centrifugal pump (2) according to claim 8 wherein the impeller (126) comprises a plurality of concentric cone-shaped rotators (146 and 148) with maximal radial ends adjacent the proximal end of the stator (136) and with minimal radial ends supported by the bearing (152).

10. A pumping unit (12; 60; 86; 124) or centrifugal pump (2) according to claim 4 wherein the magnetic means (59; 82; 110; 158) comprises an annular magnetic ring having a plurality of magnetic poles.

11. A pumping unit (12; 60; 86; 124) or centrifugal pump (2) according to claim 4 wherein the magnetic means (59; 82; 110; 158) comprises a plurality of circumferentially spaced magnets.

## Patentansprüche

1. Einmal verwendbare Pumpeinheit (12; 60; 86; 124), die lösbar an einer Magnetantriebseinrichtung (14) befestigt werden kann, um Blut zu pumpen, wobei die Pumpeinheit (12; 60; 86; 124) folgendes umfaßt:
ein Pumpengehäuse (30 und 32; 62; 92; 128) mit einer darin befindlichen Pumpkammer (P; 70) sowie einem Einlaß (18; 66; 134) und einem Auslaß (20), die mit der Pumpkammer (P; 70) in Verbindung stehen;
ein Lager (42; 78B, 98; 152), das in der Pumpkammer (P; 70) gelagert ist;
ein Flügelrad (44; 72; 90; 126), das in der Pumpkammer (P; 70) positioniert ist, wobei das Flügelrad (44; 72; 90; 126) eine Nabe (46; 80; 106) besitzt, die drehbar auf dem Lager (42; 78B; 98; 152) gelagert ist, so daß sie sich um eine Mittelachse (C) dreht; und
eine Magneteinrichtung (59; 82; 110; 158), die auf dem Flügelrad (44; 72; 90; 126) in der Pumpkammer (P; 70) angeordnet ist und mit einer Magnetantriebseinrichtung (14) magnetisch gekoppelt werden kann, um das Flügelrad (44; 72; 90; 126) in Drehung zu versetzen und auf diese Weise Flüssigkeit durch die Pumpeinheit (12; 60; 86; 124) zu pumpen, dadurch gekennzeichnet, daß:
das Flügelrad eine Vielzahl von Öffnungen aufweist, die so konfiguriert sind, daß sie den Blutstrom an dem Lager (42; 78B; 98; 152) entlangführen.

2. Zentrifugalpumpe (2) zum Pumpen von Blut, umfassend:
eine einmal verwendbare Pumpeinheit (12; 60; 86; 124), die folgendes umfaßt:
ein Pumpengehäuse (30 und 32; 62; 92; 128) mit einer darin befindlichen Pumpkammer (P; 70) sowie einem Einlaß (18; 66; 134) und einem Auslaß (20), die mit der Pumpkammer (P; 70) in Verbindung stehen;
ein Lager (42; 78B; 98; 152), das in der Pumpkammer (P; 70) gelagert ist, wobei das Lager (42; 78B; 98; 152) eine Mittelachse (C) besitzt;
ein Flügelrad (44; 72; 90; 126), das in der Pumpkammer (P; 70) positioniert ist und auf dem Lager (42; 78B; 98; 152) gelagert ist, so daß es sich um die Mittelachse (C) drehen kann, wobei das Flügelrad (44; 72; 90; 126) eine Nabe (46; 80; 106) aufweist;
eine Magneteinrichtung (59; 82; 110; 158), die auf dem Flügelrad (44; 72; 90; 126) angeordnet ist; und
eine Magnetantriebseinrichtung (14), die lösbar mit dem Pumpgehäuse (32; 62; 92; 128) verbunden werden kann, um mit der auf dem Flügelrad (44; 72; 90; 126) angeordneten Magneteinrichtung (59; 82; 110; 158) zu kommunizieren und auf diese Weise das Flügelrad (44; 72; 90; 126) in der Pumpkammer (P; 70) in Drehung zu versetzen; dadurch gekennzeichnet, daß:
das Flügelrad eine Vielzahl von Öffnungen aufweist, die so konfiguriert sind, daß sie den Blutstrom an dem Lager (42; 78B; 98; 152) entlangführen.

3. Zentrifugalpumpe (2) nach Anspruch 2, bei der die Magnetantriebseinrichtung (14) folgendes umfaßt:
einen Rotor (22), der neben dem Pumpengehäuse (32; 62; 92; 128) positioniert ist; und
eine Vielzahl von Antriebsmagneten (24), die ringförmig im Abstand am Umfang des Rotors (22) angeordnet sind und mit den magnetischen Feldlinien (F) ausgerichtet sind, die von den Antriebsmagneten (24) erzeugt werden und mit den magnetischen Feldlinien (F) fluchten, die von der auf dem Flügelrad (44; 72; 90; 126) angeordneten Magneteinrichtung (59; 82; 110; 158) erzeugt werden und die Mittelachse (C) schneiden, so daß die dabei entstehenden auf die Nabe (46; 80; 106) des Flügelrades einwirkenden Ungleichgewichtskräfte so gerichtet sind, daß sie die Nabe (46; 80; 106) des Flügelrades in Richtung zu dem Lager (42; 78B; 98; 152) drücken, wobei die dabei entstehenden Ungleichgewichtskräfte im allgemeinen parallel zu der Mittelachse (C) verlaufen und dadurch die Drehung des Flügelrades (44; 72; 90; 126) um das Lager (42; 78B; 98; 152) und die Mittelachse (C) stabilisieren.

4. Pumpeinheit (12; 60; 86; 124) oder Zentrifugalpumpe (2) nach Anspruch 1, 2 oder 3, bei der das Lager (42; 78B; 98; 152) neben dem Einlaß (18; 66; 134) gelagert ist, so daß der Strom der eingeleiteten Flüssigkeit über das Lager (42; 78B; 98; 152) geführt wird, wobei das Lager (42; 78B; 98; 152) und der Einlaß (18; 66; 134) auf der mittigen Drehachse (C) des Flügelrades (44; 72; 90; 126) ausgerichtet sind, die Richtung entlang der Mittelachse (C) von dem Lager (42; 78B; 98; 152) zu dem Einlaß (18; 66) hin die stromaufwärtige Richtung darstellt, und die Magneteinrichtung (69; 82; 110; 158) magnetische Feldlinien (F) bildet, die die mittige Drehachse (C) des Flügelrades (44; 72; 90; 126) an dem Lager (42; 78B; 98; 152) oder oberhalb desselben schneiden.

5. Pumpeinheit (12; 60; 86; 124) oder Zentrifugalpumpe (2) nach Anspruch 4, bei der das Pumpengehäuse (30 und 32; 62; 92; 128) folgendes umfaßt:
eine Gehäusekappe (30; 64; 94; 130), bei der der Einlaß (18; 66) mit der Mittelachse (C) ausgerichtet ist, um Flüssigkeit in die Pumpkammer (P; 70) zu leiten;
einen Gehäuseboden (32; 68; 96; 132), der mit der Gehäusekappe (30; 64; 94; 130) verbunden ist und auf diese Weise die Pumpkammer (P; 70) darin einschließt; und
eine ringförmige flüssigkeitsdichte Dichtung (34) zwischen der Gehäusekappe (30; 64; 94; 130) und dem Boden (32; 68; 96; 132).

6. Pumpeinheit (12; 60; 86) oder Zentrifugalpumpe (2) nach Anspruch 4, bei der das Flügelrad (44; 72; 90) einen Umfang und eine Vielzahl radialer, zu dem Umfang hin verlaufender Schaufeln (48) aufweist, wobei die Nabe (46; 80; 106) des Flügelrades (44; 72; 90) zwischen dem Einlaß (18; 66) und dem Lager (42; 78B; 98) angeordnet ist, die radialen Schaufeln (48) die Vielzahl von Öffnungen des Flügelrades (44; 72; 90) aufweisen, die so konfiguriert sind, daß das Lager (42; 78B; 98) für die von dem Einlaß (18; 66) heranströmende Flüssigkeit freiliegt, und wobei die Magneteinrichtung (59) magnetische Feldlinien (F) erzeugt, die die mittige Drehachse (c) des Flügelrades (44; 72; 90) schneiden, so daß die resultierenden, auf das Flügelrad (44; 72; 90) einwirkenden Gleichgewichts- und Ungleichgewichtskräfte die Drehung um das Lager (42; 78B; 98) und die Mittelachse (C) stabilisieren.

7. Pumpeinheit (60) oder Zentrifugalpumpe (2) nach Anspruch 5 oder 6, bei der die Pumpeinheit (60) des weiteren einen Stator (74) mit einem proximalen Ende und einem distalen Ende (78B) umfaßt, wobei das proximale Ende mit dem Pumpengehäuse (62) verbunden ist, und das distale Ende (78B) in die Pumpkammer (70) hineinragt; und wobei der Stator (74) folgendes umfaßt:
einen Statorboden (76), der als Kegelstumpf mit trapezförmigem Querschnitt entlang der Mittelachse (C) ausgebildet ist, wobei der Statorboden (76) eine maximale radiale Abmessung und eine minimale radiale Abmessung aufweist, und die maximale radiale Abmessung das proximale Ende des Stators (74) bildet; und
eine Spindel (78) mit ersten und zweiten einander entgegengesetzten Enden (78A und 78B), die mit der Mittelachse (C) ausgerichtet ist, wobei das erste Ende (78A) mit der minimalen radialen Abmessung des Statorbodens (76) verbunden ist, und das zweite Ende (78B) das distale Ende (78B) des Stators (74) bildet;
wobei das Lager (78B) einstückig mit dem zweiten Ende (78B) der Spindel (78) ausgebildet ist, die mit der Mittelachse (C) ausgerichtet ist.

8. Pumpeinheit (12; 86; 124) oder Zentrifugalpumpe (2) nach Anspruch 4 oder 5, bei der die Pumpeinheit (12; 86; 124) des weiteren einen Stator (36; 88; 136) mit einem proximalen Ende und einem distalen Ende umfaßt, wobei das proximale Ende mit dem Gehäuseboden (32; 96; 132) des Pumpengehäuses (30 und 32; 92; 128) verbunden ist, und das distale Ende in die Pumpkammer (P) hineinragt, wobei der Stator (36; 88; 136) eine mit der Drehachse (C) des Flügelrades (44; 90; 126) koaxiale Mittelachse (C) aufweist und einen proximalen Abschnitt (38; 102; 140) aus polymerem Material und einen distalen Abschnitt (40; 100; 142) aus hartbeschichtetem Aluminium besitzt, wobei der Stator (36; 88; 136) konisch geformt ist, so daß das proximale Ende einen größeren Radius besitzt als das distale Ende; wobei das Lager (42; 98; 152) ein Kugelzapfenlager (42; 98; 152) ist, das einstückig aus hartbeschichtetem Aluminium hergestellt ist, und der distale Abschnitt (40; 100; 142) des Stators (36; 88; 136) mit der Mittelachse (C) ausgerichtet ist; wobei das Flügelrad (44; 90; 126) konisch geformt ist mit einem maximalen radialen Ende im Bereich des proximalen Endes des Stators (36; 88; 136) und einem minimalen radialen Ende, das von dem Lager (42; 98; 152) aufgenommen wird.

9. Pumpeinheit (124) oder Zentrifugalpumpe (2) nach Anspruch 8, bei der das Flügelrad (126) eine Vielzahl von konzentrischen kegelförmigen Rotatoren (146 und 148) aufweist, deren maximale radiale Enden im Bereich des proximalen Endes des Stators (136) liegen, und deren minimale radiale Enden von dem Lager (152) aufgenommen werden.

10. Pumpeinheit (12; 60; 86; 124) oder Zentrifugalpumpe (2) nach Anspruch 4, bei der die Magneteinrichtung (59; 82; 110; 158) einen ringförmigen Magnetring mit einer Vielzahl von Magnetpolen aufweist.

11. Pumpeinheit (12; 60; 86; 124) oder Zentrifugalpumpe (2) nach Anspruch 4, bei der die Magneteinrichtung (59; 82; 110; 158) eine Vielzahl von im Abstand am Umfang angeordneten Magneten aufweist.

## Revendications

1. Ensemble de pompage jetable (12;60;86;124) prévu pour être monté de façon séparable sur un dispositif d'entraînement magnétique (14) pour le pompage de sang, l'ensemble de pompage (12;60;86;124) comprenant :
un corps de pompe (30 et 32:62;92;128) ayant une chambre de pompage (P;70) à l'intérieur, et une entrée (18;66;134) et une sortie (20) qui communiquent avec la chambre de pompage (P;70) ;
un palier (42;78B;98;152) supporté dans la chambre de pompage (P;70) ;
une roue (44;72;90;126) placée dans la chambre de pompage (P;70), la roue (44;72;90;126) ayant un moyeu (46;80;106) supporté de façon tournante sur le palier (42;78B;98;152) pour rotation autour d'un axe central (C); et
des moyens magnétiques (59;82;110;158) portés par la roue (44;72;90;126) à l'intérieur de la chambre de pompage (P;70),prévus pour être magnétiquement couplés avec un dispositif d'entraînement magnétique (14) de manière à faire tourner la roue (44;72;90;126) et donc à pomper le fluide à travers l'ensemble de pompage (12;60;86; 124),
caractérisé en ce que :
ladite roue comporte une pluralité d'ouvertures configurées de manière à diriger unécoulement de sang le long du palier (42;78B;98;152).

2. Pompe centrifuge (2) pour le pompage de sang, comprenant :
un ensemble de pompage jetable (12;60;86;124) comportant :
un corps de pompe (30 et 32;62;92;128) ayant une chambre de pompage (P;70) à l'intérieur, et ayant une entrée (18;66;134) et une sortie (20) qui communiquent avec la chambre de pompage (P;70);
un palier (42;78B;98;152) supporté dans la chambre de pompage (P;70), le palier (42;78B;98;152) définissant un axe central (C);
une roue (44;72;90;126) placée dans la chambre de pompage (P;70) et supportée sur le palier (42;78B;98; 152) pour rotation autour de l'axe central (C), la roue (44;72;90;126) ayant un moyeu (46;80;106);
des moyens magnétiques (59;82;110;158) portés par la roue (44;72;90;126) ; et
un dispositif d'entraînement magnétique (14) pour fixation amovible au corps de pompe (32;62;92;128) de manière à communiquer avec les moyens magnétiques (59;82;110;158) portés par la roue (44;72;90;126) et à faire ainsi tourner la roue (44;72;90;126) à l'intérieur de la chambre de pompage (P;70) ;
caractérisée en ce que :
ladite roue comporte une pluralité d'ouvertures configurées de manière à diriger un écoulement de sang le long du palier (42;78B;98;152).

3. Pompe centrifuge (2) suivant la revendication 2, dans laquelle le dispositif d'entraînement magnétique (14) comprend :
un rotor (22) placé près du corps de pompe (32; 62;92;128) ; et
une pluralité d'aimants d'entraînement (24) annulairement espacés autour de la circonférence du rotor (22) et orientés de sorte que les lignes de force magnétique (F) engendrées par les aimants d'entraînement (24) s'alignent avec les lignes de force magnétique (F) engendrées par les moyens magnétiques (59;82;110;158) portés par la roue (44;72;90;126) et coupent l'axe central (C) d'une manière telle que les forces déséquilibrées résultantes agissant sur le moyeu de roue (46;80;106) sont dans une direction qui tend à déplacer le moyeu de roue (46;80; 106) vers le palier (42;78B;98;152), les forces déséquilibrées résultantes étant sensiblement parallèles à l'axe central (C), ce qui stabilise la rotation de la roue (44; 72;90;126) autour du palier (42;78B;98;152) et de l'axe central (C).

4. Ensemble de pompage (12;60;86;124) ou pompe centrifuge (2) suivant les revendications 1,2 ou 3, dans lequel le palier (42;78B;98;152) est supporté près de l'entrée (18;66;134) pour que le fluide entrant circule sur le palier (42;78B;98;152), le palier (42;78B;98;152) et l'entrée (18;66;134) étant alignés le long de l'axe central (C) de rotation de la roue (44;72,90,126), la direction le long de l'axe central (C) allant du palier (42; 78B;98;152) vers l'entrée (18;66) définissant la direction amont, les moyens magnétiques (69;82;110;158) formant des lignes de force magnétique (F) qui coupent l'axe central (C) de rotation de la roue (44;72;90;126) à l'endroit du palier (42;78B;98;152) ou en amont de celui-ci.

5. Ensemble de pompage (12;60;86;124) ou pompe centrifuge (2) suivant la revendication 4, dans lequel le corps de pompe (30 et 32;62;92;128) comprend :
un couvercle de corps (30;64;94;130) portant l'entrée (18;66) alignée avec l'axe central (C) pour l'introduction du fluide dans la chambre de pompage (P;70) ;
une base de corps (32;68;96;132) fixée au couvercle de corps (30;64;94;130) de manière à entourer la chambre de pompage intérieure (P;70) ; et
une garniture d'étanchéité annulaire (34) entre le couvercle (30;64;94;130) et la base (32;68;96;132) du corps.

6. Ensemble de pompage (12;60;86) ou pompe centrifuge (2) suivant la revendication 4, dans lequel la roue (44;72;90) comporte une périphérie et une pluralité d'ailettes radiales (48) s'étendant jusqu'à la périphérie, le moyeu (46;80;106) de la roue (44;72;90) étant situé entre l'entrée (18;66) et le palier (42;78B;98), les ailettes radiales (48) définissant la pluralité d'ouvertures de la roue (44;72;90) qui sont configurées de manière à exposer le palier (42;78B;98) au fluide entrant venant de l'entrée (18;66), les moyens magnétiques (59) engendrant des lignes de force magnétique (F) qui coupent l'axe central (C) de rotation de la roue (44;72;90) de sorte que les forces équilibrées et déséquilibrées résultantes agissant sur la roue (44;72;90) stabilisent la rotation autour du palier (42;78B;98)et de l'axe central (C).

7. Ensemble de pompage (60) ou pompe centrifuge (2) suivant les revendications 5 ou 6, dans lequel l'ensemble de pompage (60) comprend en outre un stator (74) ayant une extrémité proximale et une extrémité distale (78B), l'extrémité proximale étant reliée au corps de pompe (62) et l'extrémité distale (78B) s'étendant dans la chambre de pompage (70), le stator (74) comprenant :
une base de stator (76) de forme tronconique à section transversale trapézoïdale le long de l'axe central (C), la base de stator (76) ayant une dimension radiale maximale et une dimension radiale minimale, la dimension radiale maximale définissant l'extrémité proximale du stator (74) ; et
une broche (78) ayant une première et une deuxième extrémité opposées (78A et 78B) alignées avec l'axe central (C), la première extrémité (78A) étant fixée à la dimension radiale minimale de la base de stator (76) et la deuxième extrémité (78B) définissant l'extrémité distale (78B) du stator (74) ;
le palier (78B) étant formé solidairement avec la deuxième extrémité (78B) de la broche (78) en alignement avec l'axe central (C).

8. Ensemble de pompage (12;86;124) ou pompe centrifuge (2) suivant les revendications 4 ou 5, dans lequel l'ensemble de pompage (12;86;124) comprend en outre un stator (36;88;136) ayant une extrémité proximale et une extrémité distale, l'extrémité proximale étant reliée à la base (32;96;132) du corps de pompe (30 et 32; 92;128) et l' extrémité distale s'étendant dans la chambre de pompage (P), le stator (36;88;136) définissant un axe central (C) coaxial à l'axe de rotation (C) de la roue (44; 90;126), le stator (36;88;136) comprenant une partie proximale (38;102;140) en matière de type polymère et une partie distale (40;100;142) en aluminium à revêtement dur, le stator (36;88;136) étant de forme conique et l'extrémité proximale ayant un plus grand rayon que l'extrémité distale, le palier (42;98;152) étant un palier de pivot sphérique (42;98;152) formé solidairement avec la partie distale (40;100;142) en aluminium à revêtement dur du stator (36;88;136) en alignement avec l'axe central (C), la roue (44;90;126) étant de forme conique avec une extrémité radiale maximale adjacente à l'extrémité proximale du stator (36;88;136) et une extrémité radiale minimale supportée par le palier (42;98;152).

9. Ensemble de pompage (124) ou pompe centrifuge (2) suivant la revendication 8, dans lequel la roue (126) comprend une pluralité d'éléments rotatifs coniques concentriques (146 et 148) ayant des extrémités radiales maximales adjacentes à l'extrémité proximale du stator (136) et des extrémités radiales minimales supportées par le palier (152).

10. Ensemble de pompage (12;60;86;124) ou pompe centrifuge (2) suivant la revendication 4,dans lequel les moyens magnétiques (59;82;110;158) sont constitués par un anneau magnétique annulaire ayant une pluralité de pôles magnétiques.

11. Ensemble de pompage (12;60;86;124) ou pompe centrifuge (2) suivant la revendication 4, dans lequel les moyens magnétiques (59;82;110;158) sont constitués par une pluralité d'aimants circonférentiellement espacés.
